(19) **Europäisches Patentamt European Patent Office Office européen des brevets**

(11) **EP 4 509 838 A1**

(12) **EUROPEAN PATENT APPLICATION**
published in accordance with Art. 153(4) EPC

(43) Date of publication:
19.02.2025 Bulletin 2025/08

(21) Application number: 23788308.7

(22) Date of filing: 10.04.2023

(51) International Patent Classification (IPC):
*G01N 33/92* (2006.01)      *C12Q 1/26* (2006.01)
*C12Q 1/34* (2006.01)      *C12Q 1/44* (2006.01)

(52) Cooperative Patent Classification (CPC):
C12Q 1/26; C12Q 1/34; C12Q 1/44; G01N 33/92

(86) International application number:
PCT/JP2023/014549

(87) International publication number:
WO 2023/199890 (19.10.2023 Gazette 2023/42)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 11.04.2022 JP 2022064966

(71) Applicant: Denka Company Limited
Tokyo 103-8338 (JP)

(72) Inventor: ITO, Yasuki
Tokyo 103-8338 (JP)

(74) Representative: Hoffmann Eitle
Patent- und Rechtsanwälte PartmbB
Arabellastraße 30
81925 München (DE)

(54) **REAGENT COMPOSITION AND KIT**

(57) A reagent composition for use as a first reagent composition in a method of quantifying a small dense LDL cholesterol (sdLDL-C) in a sample, the method including causing the first reagent composition to react to the sample, and after the causing the first reagent composition to react to the sample, causing a second reagent composition for quantifying the sdLDL-C to react, thereby quantifying cholesterol in a remaining lipoprotein, the reagent composition includes a nonionic surfactant and has cholesterol esterase activity, cholesterol oxidase activity, and sphingomyelinase activity, and a contact angle between the reagent composition and a polyethylene terephthalate (PET) base material is equal to or more than 63.0° and equal to or less than 67.0°.

EP 4 509 838 A1

## Description

TECHNICAL FIELD

The present invention relates to a reagent composition and a kit.

BACKGROUND ART

As a technology relating to a measurement method of LDL cholesterol, there is a method disclosed in Patent Document 1 (Japanese Unexamined Patent Publication No. 2000-325097). Patent Document 1 discloses a measurement method of lipoprotein cholesterol, which includes a first step of selectively subjecting HDL cholesterol to an enzyme reaction by adding an enzyme and a first surfactant to a sample containing a lipoprotein, a second step of selectively subjecting LDL cholesterol to an enzyme reaction by adding a second surfactant to the sample, and measuring a compound consumed by a reaction with the enzyme or a compound generated by the reaction in the first or second step to measure HDL cholesterol and/or LDL cholesterol. It is described that the measurement method and the measurement reagent can be provided, in which the method does not require a lipoprotein coagulant which increases a turbidity of a reaction solution, is not limited to the enzyme used, does not require a new addition of another enzyme in the step of reacting the LDL cholesterol, can quantify the LDL cholesterol easily and inexpensively, and can accurately and inexpensively measure the HDL cholesterol without forming a lipoprotein coagulation which interferes with optical measurement as necessary, therefore, the method is particularly useful in the field of clinical tests such as arteriosclerosis.

RELATED DOCUMENT

PATENT DOCUMENT

[Patent Document 1] Japanese Unexamined Patent Publication No. 2000-325097

SUMMARY OF THE INVENTION

TECHNICAL PROBLEM

The present inventor has studied the measurement of small dense LDL cholesterol (sdLDL-C) among LDL cholesterols. In this respect, in the technology disclosed in Patent Document 1, it has been found that there is room for improvement in terms of reaction specificity (specifically, correlation with a reference method) and accuracy after specimen dilution.
The present invention provides a measurement technology for sdLDL-C, which has excellent accuracy after specimen dilution and specificity.

SOLUTION TO PROBLEM

The present inventor has studied to improve reaction specificity (specifically, correlation with a reference method) of quantification of sdLDL-C and accuracy after specimen dilution. As a result, it was newly found that by setting a contact angle of a reagent composition used for the quantification of the sdLDL-C within a specific range, the correlation with a reference ultracentrifugation method is good, and the quantification of the sdLDL-C in which the accuracy after the specimen dilution is excellent can be performed, thereby completing the present invention.
According to the present invention, there are provided the following reagent composition and kit.

[1] A reagent composition for use as a first reagent composition in a method of quantifying a small dense LDL cholesterol (sdLDL-C) in a sample, the method including

causing the first reagent composition to react to the sample, and
after the causing the first reagent composition to react to the sample, causing a second reagent composition for quantifying the sdLDL-C to react, thereby quantifying cholesterol in a remaining lipoprotein,
the reagent composition comprising a nonionic surfactant and having cholesterol esterase activity, cholesterol oxidase activity, and sphingomyelinase activity, wherein:

a contact angle between the reagent composition and a polyethylene terephthalate (PET) base material, which is measured by the following method 1, is equal to or more than 63.0° and equal to or less than 67.0°.

(method 1)

(1) PET base material: PET (amorphous polyester) resin plate, transparent, thickness of 2 mm,
(2) Pre-treatment: a surface of the unused PET base material is wiped with a 70% ethanol aqueous solution, and a measurement is performed within 5 minutes after the wiping,
(3) A measurement method and conditions: a liquid droplet method, a θ/2 method, a temperature: 15°C to 25°C, a syringe: Teflon (registered trademark; the same applies hereinafter)-coated 18G, a dropwise addition method, a dropwise addition amount: 2 μL, a measurement 1 second after the dropwise addition, and
(4) Calculation of the contact angle: the measurement is performed 5 times, and an average value of the 5 times is obtained.

[2] The reagent composition according to [1],

in which a viscosity of the reagent composition at 5°C, which is measured by the following method 2-1, is equal to or less than 2.5 mPa·s.
(method 2-1)

(1) Device: electro magnetically spinning (EMS) viscometer,
(2) a measurement method and conditions: a measurement type: an electromagnetic spinning method, a motor rotation speed: 1,000 rpm, a holding time: 600 seconds, a measurement temperature: 5°C, a sequence loop: 1 time, a sample: 500 μL, and
(3) Calculation of the viscosity: the measurement is performed 5 times, and an average value of the 5 times is calculated.

[3] The reagent composition according to [1] or [2],

in which a viscosity of the reagent composition at 37°C, which is measured by the following method 2-2, is equal to or less than 1.05 mPa·s.
(method 2-2)

(1) Device: electro magnetically spinning (EMS) viscometer,
(2) A measurement method and conditions: a measurement type: an electromagnetic spinning method, a motor rotation speed: 1,000 rpm, a holding time: 600 seconds, a measurement temperature: 37°C, a sequence loop: 1 time, a sample: 500 μL, and
(3) Calculation of the viscosity: the measurement is performed 5 times, and an average value of the 5 times is calculated.

[4] The reagent composition according to any one of [1] to [3],
in which the nonionic surfactant contains polyoxyethylene monostyrenated phenyl ether.
[5] The reagent composition according to [4],
in which a degree of polymerization n of polyoxyethylene in the polyoxyethylene monostyrenated phenyl ether is equal to or more than 5 and equal to or less than 80.
[6] The reagent composition according to [4] or [5],
in which a content of the polyoxyethylene monostyrenated phenyl ether in the reagent composition is equal to or more than 0.05% (w/v) and equal to or less than 0.6% (w/v) with respect to an entire reagent composition.
[7] The reagent composition according to any one of [1] to [6],
in which the reagent composition contains any one of a hydrogen donor or a coupler.
[8] The reagent composition according to any one of [1] to [7],
in which the reagent composition further has ascorbic acid oxidase activity.
[9] The reagent composition according to any one of [1] to [8],
in which the reagent composition further has at least one activity selected from the group consisting of peroxidase activity and catalase activity.
[10] A kit used for quantifying a sdLDL-C in a sample, the kit containing

a first reagent composition consisting of the reagent composition according to any one of [1] to [9], and
a second reagent composition for quantifying the sdLDL-C.

[11] The kit according to [10],
in which the second reagent composition has peroxidase activity.

ADVANTAGEOUS EFFECTS OF INVENTION

According to the present invention, it is possible to provide a measurement technology for sdLDL-C, which has excellent reaction specificity (specifically, correlation with a reference method) and accuracy after specimen dilution.

BRIEF DESCRIPTION OF THE DRAWINGS

[Fig. 1] Diagrams showing an evaluation result of accuracy after specimen dilution of a measurement value of sdLDL-C.
[Fig. 2] Diagrams showing an evaluation result of reaction specificity (correlation with a reference method) of the measurement value of the sdLDL-C.
[Fig. 3] A diagram showing a relationship between the reaction specificity (a correlation coefficient in the correlation with the reference method) of the measurement value of the sdLDL-C and the contact angle.
[Fig. 4] Diagrams showing an evaluation result of dilution linearity of the measurement value of the sdLDL-C.
[Fig. 5] Diagrams showing the evaluation result of the dilution linearity of the measurement value of the sdLDL-C.

DESCRIPTION OF EMBODIMENTS

Hereinafter, embodiments will be described. In the present embodiment, the composition such as the measurement reagent can contain each component alone or in combination of two or more. In addition, in the present specification, a numerical range "x to y" indicates "equal to or more than x and equal to or less than y", and includes both the lower limit value x and the upper limit value y.
First, a lipoprotein will be described.
A lipoprotein can be fractionated roughly into very low density lipoprotein (VLDL), low density lipoprotein (LDL) and high density lipoprotein (HDL), and LDL is further sub-fractionated into a small dense LDL (sdLDL), and other sub-fractions. The sdLDL is also referred to as small particle LDL; small LDL (SLDL); dense LDL; small, dense LDL, and LDLs other than the above-described sdLDL are sometimes referred to as large LDL (L LDL) or light LDL.
These lipoprotein fractions and sub-fractions can be distinguished based on a particle size or a density (specific gravity).
The particle size of lipoprotein in diameter is, 30 nm to 80 nm (or 30 nm to 75 nm) for VLDL, 22 nm to 28 nm (or 19 nm to 30 nm) for LDL, and 7 nm to 10 nm for HDL, although the figures may vary depending on the researchers.
The density of the lipoprotein is, for example, equal to or less than 1.006 for VLDL, 1.019 to 1.063 for LDL, and 1.063 to 1.21 for HDL.
Among lipoproteins, the diameter of LDL particles can be measured by, for example, gradient gel electrophoresis (GGE) (JAMA, 260, p. 1917 to 21, 1988) or NMR (HANDBOOK OF LIPOPROTEIN TESTING 2nd Edition, Edited by Nader Rifai et al., p. 609 to 623, AACC PRESS: The Fats of Life Summer 2002, LVDD 15 YEAR ANNIVERSARY ISSUE, Volume AVI No. 3, p. 15 to 16). The specific gravity can be determined based on, for example, analysis by ultracentrifugation (Atherosclerosis, 106, p. 241 to 253, 1994: Atherosclerosis, 83, p. 59, 1990).
In the present embodiment, the sdLDL to be measured generally refers to a sub-fraction having a diameter of approximately 22.0 to approximately 25.5 nm or a sub-fraction having a density of 1.040 to 1.063 among LDL fractions.
The reason why LDL is sub-fractionated according to the particle size is that a small LDL among LDLs needs to be fractionally measured since LDL with a small particle diameter causes more arteriosclerosis and is higher in malignancy than other LDLs. Since the distributions of diameter and density of an LDL are continuous, it is not possible to clearly distinguish the value of density above which the malignancy is notably higher. Therefore, the dense value of 1.040 to 1.063 described above is not an established characteristic of sdLDL, but is a value on the high dense side obtained by dividing the dense range of 1.019 to 1.063, which is widely used and established as the density of LDL, at a median point. For example, regarding the density of sdLDL, in a different report, sdLDL is fractionated in a range of 1.044 to 1.060 (Atherosclerosis: 106 241-253 1994). There are some differences among researchers on how to set the range of the density of sdLDL, but with any of the ranges fractionated, the presence of sdLDL is associated with clinical malignancy.
In the present specification, in a case where sdLDL is referred to, it specifically refers to an LDL that has a higher density among LDLs and is clinically more arteriosclerogenic than other LDLs. In addition, sdLDL preferably refers to an LDL having a density belonging to a dense range higher than the median point in the dense range of LDLs and more preferably having a density belonging to a range of 1.044 to 1.063. Further, lipoproteins other than LDL may refer to VLDL or HDL, or may further include chylomicrons and intermediate density lipoproteins (IDL).

The present inventor has conducted studies to improve the accuracy when quantifying sdLDL-C in a sample. As a result, it has been found that when sdLDL cholesterol (sdLDL-C) is quantified by a method including causing the first reagent composition to react to the sample (first step), and then causing a second reagent composition for quantifying the sdLDL-C to react, thereby quantifying cholesterol in a remaining lipoprotein (second step), in the first step, it is important to highly control the selectivity of the action of the first reagent composition on LDLs other than sdLDL. More specifically, in the first step, it is important that the first reagent composition selectively acts on LDLs other than sdLDL, and when the selectivity is too low, that is, the first reagent composition is a reagent that easily acts on sdLDL as a measurement target, there is a concern that the accuracy of the quantification of sdLDL-C in the sample in the second step may be decreased.

Therefore, the present inventor has further studied to set the selectivity of the action of the first reagent composition to be more preferable and has found that, by configuring the first reagent composition to have specific enzyme activity and to have a contact angle within a specific range, the first reagent composition which acts on the LDLs other than the sdLDL with high selectivity and makes it possible to quantify the sdLDL-C with excellent reaction specificity (correlation with the reference method) and excellent accuracy after the specimen dilution, can be stably obtained.

In the present embodiment, since the first reagent composition contains specific components and has a contact angle in a specific range, the first reagent composition can be suitably used for quantifying sdLDL-C and can have excellent accuracy in the measurement of sdLDL-C.

For example, the first reagent composition is used in combination with the second reagent composition described later for quantifying the sdLDL-C.

Hereinafter, each reagent composition will be described in more detail.

(Reagent composition (first reagent composition))

In the present embodiment, the reagent composition is for use as a first reagent composition in a method of quantifying a small dense LDL cholesterol (sdLDL-C) in a sample, the method including causing the first reagent composition to react to the sample, and after the causing the first reagent composition to react to the sample, causing a second reagent composition for quantifying the sdLDL-C to react, thereby quantifying cholesterol in a remaining lipoprotein.

Hereinafter, the reagent composition used as the first reagent composition is also simply referred to as a "first reagent composition".

The first reagent composition contains a nonionic surfactant and has cholesterol esterase activity, cholesterol oxidase activity, and sphingomyelinase activity. A contact angle between the first reagent composition and a polyethylene terephthalate (PET) base material, which is measured by the following method 1, is equal to or more than 63.0° and equal to or less than 67.0°.

(Method 1)

(1) PET base material: PET (amorphous polyester) resin plate, transparent, thickness of 2 mm,
(2) Pre-treatment: a surface of the unused PET base material is wiped with a 70% ethanol aqueous solution, and a measurement is performed within 5 minutes after the wiping,
(3) Measurement method and conditions: a liquid droplet method, a $\theta/2$ method, a temperature: 15°C to 25°C, a syringe of Teflon-coated 18G, a dropwise addition method, a dropwise addition amount: 2 $\mu$L, a measurement 1 second after the dropwise addition, and
(4) Calculation of the contact angle: the measurement is performed 5 times, and an average value of the 5 times is obtained.

In the above (2), dust or the like on the PET base material is wiped off before the measurement. Since electrostatics affects the contact angle, the PET base material is wiped off as much as possible so that friction or the like does not occur, and then the measurement is performed within 5 minutes.

In the present embodiment, the contact angle is specifically a static contact angle measured by, for example, DMo-901, 701, 601, 501, DMC-MC3, or the like manufactured by Kyowa Interface Science Co., Ltd.

The present inventor has newly found that, by setting the contact angle between the first reagent composition and the PET base material within the specific range in the measurement reagent of the sdLDL-C, the quantification of the sdLDL-C in which reaction specificity (correlation with the reference method) and accuracy after specimen dilution are excellent can be performed. The reason for this is not always clear, but the following is considered. In the quantification of the sdLDL-C, for example, in the first step, the first reagent composition eliminates cholesterol in lipoproteins other than the sdLDL. In this case, the contact angle may be related to the accuracy after the specimen dilution. The reason is not clear, but when the specimen is diluted, a protein component or a measurement target (sdLDL) contained in the specimen is diluted. Therefore, when the specimen is mixed with a first reagent product, the sdLDL may erroneously react due to the influence of the dilution, so that the sdLDL-C may be eliminated. By controlling the contact angle, the erroneous reaction is suppressed. Therefore, it is considered that the sdLDL-C can be more accurately assumed. In addition, in a case where the contact angle is outside the specific range, it is expected that, even in an undiluted specimen, when the first reagent composition is mixed with the specimen in the first step, reaction of the lipoproteins contained in the specimen with the nonionic surfactant or a specific enzyme in the first reagent composition changes. It is considered that an accurate measurement of the sdLDL-C is difficult due to the erroneous reaction, and the correlation with the reference method related to the reaction specificity is reduced. Here, the reference method is specifically an ultracentrifugation method.

From the viewpoint of further improving the reaction specificity (the correlation with the reference method) of the measurement of the sdLDL-C and the accuracy after the specimen dilution, the contact angle between the reagent composition and the PET base material is equal to or more than 63.0°, and is preferably equal to or more than 63.8°.

In addition, from the viewpoint of further improving the reaction specificity (the correlation with the reference method) of the measurement of the sdLDL-C, the contact angle between the reagent composition and the PET base material is equal to or less than 67.0°, is preferably equal to or less than 66.5°, and is more preferably equal to or less than 66.0°.

In the present embodiment, for example, the contact angle with the PET base material can be controlled by appropriately selecting the type, combination, blending amount, and the like of each component contained in the first reagent composition. Among these, for example, adjustment of the type and blending amount of a surfactant, the type and blending amount of an enzyme or a protein, and the like are shown an example of factors for setting the contact angle with the PET base material in a desired numerical range.

(Viscosity)

The property of the first reagent composition is specifically liquid.

In a viscosity of the first reagent composition at 5°C, from the viewpoint of improving the accuracy of the measurement, the viscosity of the first reagent composition at 5°C may be, for example, equal to or less than 2.5 mPa·s, is preferably equal to or less than 2.2 mPa·s, and is more preferably equal to or less than 2.0 mPa·s.

In addition, the viscosity of the first reagent composition at 5°C may be, for example, equal to or more than 1.6 mPa·s, or may be, for example, equal to or more than 1.8 mPa·s.

From the viewpoint of improving the accuracy of the measurement, a viscosity of the first reagent composition at 37°C is preferably equal to or less than 1.05 mPa·s, more preferably equal to or less than 1.00 mPa·s, and still more preferably equal to or less than 0.95 mPa·s.

In addition, a viscosity of the first reagent composition at 37°C may be, for example, equal to or more than 0.80 mPa·s, or may be, for example, equal to or more than 0.90 mPa·s.

Here, the viscosity of the first reagent composition at each temperature is measured by the following method 2. In the method 2, a method in which the measurement temperature is set to 5°C is a method 2-1, and a method in which the measurement temperature is set to 37°C is a method 2-2.

(Method 2)

(1) Device: electro magnetically spinning (EMS) viscometer,
(2) Measurement method and conditions: a measurement type: an electromagnetic spinning method, a motor rotation speed: 1,000 rpm, a holding time: 600 seconds, a measurement temperature: 5°C (method 2-1) and 37°C (method 2-2), a sequence loop: 1 time, a sample: 500 μL, and
(3) Calculation of the viscosity: the measurement is performed 5 times, and an average value of the 5 times is calculated.

In the present embodiment, the viscosity is measured by, for example, EMS-1000 manufactured by KYOTO ELECTRONICS MANUFACTURING Co., Ltd.

In addition, among measurement reagents for sdLDL-C, the present inventor has newly found that, by setting the viscosity of the first reagent composition in a specific range, it is possible to quantify the sdLDL-C with more excellent accuracy. The reason for this is not always clear, but the following is considered. In the quantification of the sdLDL-C, when the measurement is performed, for example, a reagent probe sucks a certain amount of the first reagent composition to discharge the first reagent composition into a reaction cell. In a case where the viscosity of the first reagent composition used in eliminating the cholesterol in the lipoproteins other than sdLDL is too high, a discharge amount may change due to the viscosity, and the eliminated amount may fluctuate. However, since the liquid separation of the reagent can be appropriately controlled by accurately controlling the viscosity, it is considered that as a result, the sdLDL-C can be measured more accurately.

Since the first reagent composition is that contains the nonionic surfactant and has specific enzyme activity, when the first reagent composition is added to the sample, for example, the first reagent composition can act on the lipoproteins other than the sdLDL in the sample, so that elimination can be performed. In addition, the cholesterol in the lipoproteins other than the sdLDL, notably cholesterol in lbLDL can be stably led to an outside of a reaction system.

Here, the expression "surfactant acts (reacts)" refers to that a surfactant degrades lipoproteins to liberate cholesterol in the lipoproteins. For example, in the case of "surfactant that acts on (reacts with) lipoproteins other than sdLDL", it is not required that the surfactant does not act on sdLDL at all, and the surfactant may mainly act on lipoproteins other than sdLDL. Similarly, in the case of "surfactant that acts on (reacts with) LDLs other than sdLDL", the surfactant may mainly act on LDLs other than sdLDL. The term "elimination" means that a substance in a test sample is degraded to prevent the degraded product from being detected in the subsequent step. That is, the expression "eliminating cholesterol in lipoproteins other than sdLDL" means that lipoproteins other than sdLDL in the test sample are degraded to prevent the cholesterol in the lipoproteins, which is a degraded product, from being detected in the subsequent step. Similarly, the expression "eliminating cholesterol in LDLs other than sdLDL" means that cholesterol in LDLs other than sdLDL in the test sample is prevented from being detected in the subsequent step.

Specifically, the expression "led to the outside of the reaction system" means that cholesterol contained in HDL, VLDL, L LDL, and the like is eliminated or aggregated together to prevent the cholesterol contained in HDL, VLDL, L LDL, and the like from affecting the quantification of sdLDL-C, or the cholesterol contained in HDL, VLDL, L LDL, and the like is inhibited to avoid the reaction thereof in the subsequent step.

Hereinafter, the components contained in the first reagent composition will be described in more detail.

(Surfactant)

The first reagent composition contains at least a nonionic surfactant. The surfactant is specifically a surfactant which acts on the lipoproteins other than sdLDL, and more preferably a surfactant which acts on the lipoproteins other than sdLDL and does not act on the sdLDL.

From the viewpoint of further improving the accuracy after the specimen dilution in the measurement of the sdLDL-C and the specificity, the nonionic surfactant is preferably a polyoxyethylene derivative, more preferably a polyoxyethylene polycyclic phenyl ether derivative, still more preferably one or two selected from the group consisting of a polyoxyethylene benzylphenyl ether derivative and a polyoxyethylene styrenated phenyl ether derivative, and even more preferably includes a polyoxyethylene monostyrenated phenyl ether (POE monostyrenated phenyl ether). From the same viewpoint, it is also preferable that the nonionic surfactant includes a polyoxyethylene benzylphenyl ether derivative and a polyoxyethylene styrenated phenyl ether derivative.

The POE monostyrenated phenyl ether may be composed of a plurality of compounds having different degrees of polymerization in POE moieties. In addition, from the viewpoint of improving the accuracy in quantification of sdLDL-C, the average degree of polymerization of oxyethylene is, for example, 1 or more, preferably equal to or more than 5, and more preferably equal to or more than 10, and for example, equal to or less than 100, preferably equal to or less than 80, more preferably equal to or less than 50, and still more preferably equal to or less than 40, and may be, for example, equal to or less than 30.

Here, the average degree of polymerization is obtained from the intensity of the oxyethylene signal of the POE monostyrenated phenyl ether measured by NMR. For example, the average degree of polymerization can be calculated from a ratio of the signal intensity of the methylene protons (4 pieces) of the POE moiety of 3.5 ppm based on the intensity of the protons (9 pieces) of the TMS group of 0.08 ppm in the NMR spectrum of the TMS derivative of the POE monostyrenated phenyl ether.

In addition, from the viewpoint of more stably improving the accuracy in quantification of sdLDL-C, it is preferable that one or more compounds selected from the group consisting of compounds in which the average degree of polymerization n of oxyethylene in the POE monostyrenated phenyl ether is n = 10 to 20 are contained.

Here, the degree of polymerization of oxyethylene of the component constituting the POE monostyrenated phenyl ether is obtained by structural analysis by liquid chromatograph mass spectrometry. For example, when the POE monostyrenated phenyl ether in the first reagent composition is separated by a liquid chromatograph (LC) and then is introduced into a quadrangular time-of-flight mass spectrometer (QTOF mass spectrometer), for each component, a mass spectrum with peaks having an interval of 44 amu is obtained due to the difference in the degree of polymerization of oxyethylene. The degree of polymerization of oxyethylene of each component can be calculated by subtracting the mass number (MW 198) of monostyrenated phenol from the molecular weight estimated from the m/z of each peak and dividing the obtained value by 44. Further, the degree of polymerization of oxyethylene of each component can be analyzed by subtracting the chemical composition ($C_{14}H_{14}O$) of monostyrenated phenol from the compositional formula obtained from the accurate mass for each peak, or carrying out a detailed structural analysis by carrying out product ion scan.

From the viewpoint of improving the accuracy in quantification of sdLDL-C, the content of the POE monostyrenated phenyl ether in the first reagent composition is preferably equal to or more than 0.01% (w/v), more preferably equal to or more than 0.03% (w/v), still more preferably equal to or more than 0.05% (w/v), and even still more preferably equal to or more than 0.1% (w/v) with respect to the entire first reagent composition.

In addition, from the same viewpoint, the content of the POE monostyrenated phenyl ether in the first reagent composition is preferably equal to or less than 0.6% (w/v), more preferably equal to or less than 0.4% (w/v), still more preferably equal to or less than 0.35% (w/v), and even still more preferably equal to or less than 0.3% (w/v) with respect to the entire first reagent composition.

Here, the POE monostyrenated phenyl ether is available as an industrial raw material or a research reagent, and for example, a commercially available product can be used.

In addition, for example, POE monostyrenated phenyl ether may be produced and used. Specifically, the POE monostyrenated phenyl ether can be produced according to the following method and conditions.

That is, the POE monostyrenated phenyl ether is obtained by adding a predetermined amount of ethylene oxide to monostyrenated phenol in the presence of a basic catalyst. Examples of the basic catalyst include sodium hydroxide, potassium hydroxide, and alcoholate of an alkali metal. The addition reaction temperature is preferably 120°C to 200°C.

On the other hand, a styrenated phenol is obtained by carrying out an alkylation reaction of phenol and styrene at a temperature of 70°C to 200°C in the presence of an acid catalyst. As the acid catalyst, inorganic acids such as sulfuric acid and phosphoric acid, organic acids such as p-toluenesulfonic acid and methanesulfonic acid, Lewis acids such as a boron trifluoride diethyl ether complex, and the like can be used. In this reaction, by controlling conditions such as the equivalent ratio of phenol and styrene, the type of the acid catalyst, the reaction temperature, and the like, a monostyrenated phenol in which one styrene binds to the phenol and benzene ring can be obtained.

The monostyrenated phenol can be synthesized by, for example, adding styrene dropwise to phenol in the presence of a phosphoric acid catalyst to carry out an alkylation reaction, and then adding a sulfuric acid or magnesium sulfate catalyst in order to remove unreacted phenols and styrenes to complete the alkylation reaction. At this time, the amount of the phosphoric acid catalyst used is preferably 0.001 to 0.01 equivalents with respect to the phenol. The amount of the sulfuric acid or magnesium sulfate catalyst used is preferably 2% by mass to 10% by mass based on the mass of the phosphoric acid catalyst. In addition, the equivalent ratio of phenol and styrene is preferably 0.9 to 1.3 in terms of an equivalent ratio of the styrene with respect to the phenol.

Subsequently, after an end of alkylation reaction, an aqueous solution such as sodium carbonate, potassium carbonate, sodium hydroxide, potassium hydroxide, or the like is added to neutralize the product of the alkylation reaction, and the produced neutralized salt is removed by filtration. Thus, the monostyrenated phenol can be recovered.

Then, in a pressure resistant container such as an autoclave, the POE monostyrenated phenyl ether can be obtained by carrying out an addition reaction of the obtained monostyrenated phenol and ethylene oxide under heating and pressure using potassium hydroxide as a catalyst. The heating and pressing conditions are, for example, a pressure of approximately 1.5 kg/cm³ and a temperature of approximately 130°C. In addition, by adjusting the molar ratio of ethylene oxide with respect to 1 mol of monostyrenated phenol, the number of repeating units of the oxyethylene unit, that is, the degree of polymerization of oxyethylene can be adjusted.

In addition, in the above method, a mixture of a monostyrenated phenol, a distyrenated phenol to which two styrenes are bound, and a tristyrenated phenol to which three styrenes are bound is obtained as the styrenated phenol, and the monostyrenated phenol can be separated and purified from the mixture and used.

The POE monostyrenated phenyl ether in the first reagent composition can be confirmed by, for example, a method of carrying out analysis by combining IR, NMR, LC-MS and the like. Examples of a method of determining the structure of the POE monostyrenated phenyl ether in the first reagent composition include method of carrying out analysis using LC/MS/MS and NMR.

In addition, the concentration of the POE monostyrenated phenyl ether in the first reagent composition can be calculated, for example, by HPLC or LC/MS measurement. In addition, the concentration of the POE monostyrenated phenyl ether in the first reagent composition can be obtained by NMR measurement.

From the viewpoint of further improving the dilution linearity and the accuracy after the specimen dilution by stably acting the nonionic surfactant on the lipoproteins other than the sdLDL, a concentration of the nonionic surfactant in the first reagent composition is preferably equal to or more than 0.01% (w/v), more preferably equal to or more than 0.025% (w/v), still more preferably equal to or more than 0.03% (w/v), even more preferably equal to or more than 0.05% (w/v), much more preferably equal to or more than 0.075% (w/v), still much more preferably equal to or more than 0.1% (w/v), far more preferably equal to or more than 0.125% (w/v), and still far more preferably equal to or more than 0.25% (w/v) with respect to a total composition of the first reagent composition.

In addition, the concentration of the nonionic surfactant in the first reagent composition is preferably equal to or less than 1.5% (w/v), more preferably equal to or less than 1.0% (w/v), still more preferably equal to or less than 0.875% (w/v), even more preferably equal to or less than 0.75% (w/v), even still more preferably equal to or less than 0.6% (w/v), and further more preferably equal to or less than 0.3% (w/v) with respect to the total composition of the first reagent composition.

The nonionic surfactant in the first reagent composition can be identified by, for example, a method of analyzing in combination with IR, NMR, LC-MS, and the like. Examples of a method for determining a structure of the nonionic surfactant in the first reagent composition include a method of analyzing the nonionic surfactant using LC-MSMS and NMR.

(Enzyme)

The first reagent composition has cholesterol esterase (CHE) activity, cholesterol oxidase (COO) activity, and sphingomyelinase (SMase) activity. As a result, the first reagent composition can be stably acted on by the lipoproteins other than sdLDL to lead the cholesterol to the outside of the reaction system.

In addition, the first reagent composition specifically contains an enzyme having cholesterol esterase activity, an enzyme having cholesterol oxidase activity, and an enzyme having sphingomyelinase activity. The first reagent composition further specifically contains cholesterol esterase, cholesterol oxidase, and sphingomyelinase.

As the cholesterol esterase, the cholesterol oxidase, the peroxidase, and the catalase, it is possible to use, for example, those derived from bacteria or fungi, or those derived from plants.

Here, the expression "has cholesterol esterase activity" specifically means that cholesterol esterase is present and a reaction catalyzed by the cholesterol esterase may occur. The same applies to other enzyme activities such as cholesterol oxidase activity.

From the viewpoint of more stably leading cholesterol in lipoproteins other sdLDL to the outside of the reaction system, the cholesterol esterase activity of the first reagent composition is preferably equal to or more than 50 U/L and more preferably equal to or more than 100 U/L, and is preferably equal to or less than 3000 U/L, more preferably equal to or less than 2500 U/L, still more preferably equal to or less than 2000 U/L, even still more preferably equal to or less than 1000 U/L. The cholesterol esterase activity may be, for example, equal to or less than 1800 U/L, or, for example, equal to or less than 1500 U/L.

From the same viewpoint, the cholesterol oxidase activity of the first reagent composition is preferably equal to or more than 100 U/L, more preferably equal to or more than 150 U/L, and preferably equal to or less than 800 U/L, more preferably equal to or less than 750 U/L, still more preferably equal to or less than 700 U/L, even more preferably equal to or less than 650 U/L, and even still more preferably equal to or less than 600 U/L.

Since the first reagent composition has the sphingomyelinase activity, in liberating cholesterol from the lipoproteins other than the sdLDL to lead the cholesterol to the outside of the reaction system (the first step described later), reactivity with the lipoproteins other than the sdLDL, specifically with L LDL, can be improved.

From the viewpoint of more stably leading cholesterol in lipoproteins other sdLDL to the outside of the reaction system, the sphingomyelinase activity of the first reagent composition is preferably equal to or more than 100 U/L and more preferably equal to or more than 200 U/L, and is preferably equal to or less than 3000 U/L and more preferably equal to or less than 2800 U/L.

From the viewpoint of more stably leading the cholesterol in the lipoproteins other than the sdLDL to the outside of the reaction system, the first reagent composition preferably further has at least one activity selected from the group consisting of peroxidase (POD) activity and catalase activity. The first reagent composition preferably further contains at least one enzyme selected from the group consisting of an enzyme having peroxidase activity and an enzyme having catalase activity, more preferably further contains at least one of peroxidase or catalase, and still more preferably further contains catalase.

From the viewpoint of more stably leading cholesterol in lipoproteins other sdLDL to the outside of the reaction system, the peroxidase activity of the first reagent composition is preferably equal to or more than 200 U/L and more preferably equal to or more than 300 U/L, is preferably equal to or less than 3000 U/L, more preferably equal to or less than 2500 U/L, and is also preferably, for example, equal to or less than 2000 U/L. In addition, the peroxidase activity of the first reagent composition may be, for example, equal to or less than 5000 U/L, for example, or equal to or less than 4000 U/L.

From the viewpoint of stably eliminating hydrogen peroxide generated when the first reagent composition is applied to the sample, and the viewpoint of more stably leading cholesterol in lipoproteins other sdLDL in the sample to the outside of the reaction system, the catalase activity of the first reagent composition is preferably equal to or more than 100 KU/L and more preferably equal to or more than 200 KU/L, and is preferably equal to or less than 2000 KU/L and more preferably equal to or less than 1500 KU/L.

In the first reagent composition, each of the cholesterol oxidase activity, cholesterol esterase activity, sphingomyelinase activity, peroxidase activity, and catalase activity can be measured by, for example, the following method.

In the measurement of the cholesterol oxidase activity, a 6 mM cholesterol solution (the cholesterol is dissolved in isopropanol) is used as a substrate solution. A dilution solution (0.1 M phosphate buffer solution, Triton X100, pH 7.0) is added so that the concentration of the measurement target is 2 to 4 U/mL, 3 mL of the diluted solution is heated at 37°C for 5 minutes, and 0.05 mL of a substrate solution is then added thereto. Then, the mixed solution is allowed to react at 37°C and the amount of change in absorbance at a wavelength of 240 nm is measured. After the reaction at 37°C, the amount of change in absorbance is measured from 2 minutes to 7 minutes, and the cholesterol oxidase activity is calculated. For example, in a case where the amount of change in absorbance is equal to or more than 3 U/L, it can be said that the measurement target has cholesterol oxidase activity, and more specifically, it can be said that cholesterol oxidase is contained.

In the measurement of the cholesterol esterase activity, a substrate (a solution of 0.04% cholesterol linolenic acid, 1% Triton X100, and 0.6% sodium cholate), a solution of 300 U/mL cholesterol oxidase, an enzyme dilution solution (a 20 mM phosphate buffer solution containing 0.5 mM EDTA·2Na, 2 mM $MgCl_2$, and 0.2% bovine serum albumin (BSA), pH 7.5), and a reaction solution (0.06% 4-aminoantipyrine, 0.4% phenol, 7.5 KU/L peroxidase (POD)) are used. After mixing 1.75 mL of the reaction solution and 1.0 mL of the substrate solution, the mixed solution is heated at 37°C for 5 minutes, and a 0.1 mL cholesterol oxidase solution is added thereto. After heating the mixed solution at 37°C for 2 minutes, 0.1 mL of the measurement target diluted with the dilution solution is added, the mixed solution is allowed to react at 37°C, and the amount of change in absorbance at a wavelength of 500 nm is measured. After the reaction at 37°C, the amount of change in the absorbance from 0 minutes to 3.5 minutes is measured, and the cholesterol esterase activity is calculated. For example, when the amount of change in the absorbance is equal to or more than 8 U/L, it can be said that the measurement target has cholesterol esterase activity, and more specifically, it can be said that cholesterol esterase is contained.

The sphingomyelinase activity of the first reagent composition is measured, for example, by the following method. That is, a reaction solution (a mixed solution of 0.008% sphingomyelin, a 0.05% Triton X100 solution, 10 U/mL alkaline phosphatase, 10 U/mL cholesterol oxidase, 2 U/mL peroxidase, 0.02% 4-aminoantipyrine, and 0.02% TODB), a reaction stopping solution (a 1% sodium dodecyl sulfate solution), and a dilution solution (a 10 mM Tris buffer solution containing 0.1% Triton X100, pH 8.0) are used. 0.08 mL of the reaction solution and 0.003 mL of the measurement target diluted with the dilution solution are mixed, the mixed solution is heated at 37°C for 5 minutes, and then 0.16 mL of the reaction stopping solution is added thereto. After stopping the reaction, the amount of change in the absorbance at a main wavelength of 546 nm and a minor wavelength of 700 nm is measured to calculate the sphingomyelinase activity. For example, in a case where the amount of change in absorbance is equal to or more than 2 U/L, it can be said that the measurement target has sphingomyelinase activity, and more specifically, it can be said that the measurement target contains sphingomyelinase.

The peroxidase activity of the first reagent composition is measured, for example, by the following method. That is, a reaction solution 1 (1.5 mM HDAOS, 0.05% Triton X100, and 50 mM phosphate buffer solution, pH 7.0) and reaction solution 2 (5 mM 4-aminoantipyrine, 0.05% Triton X100, 1% hydrogen peroxide, and 50 mM phosphate buffer solution, pH 7.0), and a dilution solution (50 mM phosphate buffer solution, pH 7.0) are used. 0.3 mL of the reaction solution 1 and 0.08 mL of the measurement target diluted with the dilution solution are mixed and the mixed solution is heated at 37°C for 5 minutes. Then, 0.1 mL of the reaction solution 2 is added thereto, the resultant mixture is allowed to react at 37°C, and the amounts of change in the absorbance at a main wavelength of 600 nm and a minor wavelength of 700 nm are measured. After the reaction at 37°C, the amount of change in the absorbance from 2 minutes to 5 minutes is measured to calculate the peroxidase activity. For example, in a case where the amount of change in the absorbance is equal to or more than 10 U/L, it can be said that the measurement target has peroxidase activity, and more specifically, it can be said that the measurement target contains peroxidase.

The catalase activity of the first reagent composition is measured, for example, by the following method. That is, in the measurement of the catalase activity, a substrate (0.06% hydrogen peroxide, and 50 mM phosphate buffer solution, pH 7.0) is used. After preheating 2.0 mL of the substrate solution at 25°C, the substrate solution is mixed with 0.1 mL of a measurement target, and the amount of change in the absorbance at 240 nm is measured. For example, after the reaction at 25°C, the amount of change in the absorbance from 0 minutes to 3 minutes is measured to calculate the catalase activity. For example, in a case where the amount of change in the absorbance is equal to or more than 50 U/L, it can be said that the measurement target has a catalase activity, and more specifically, it can be said that the measurement target contains catalase.

The first reagent composition may further have other enzyme activities, and specifically, one or two or more enzyme activities selected from the group consisting of ascorbic acid oxidase activity and lipoprotein lipase (LPL) activity.

In addition, the first reagent composition may contain, for example, an enzyme having one or two or more enzyme activities selected from the group consisting of an enzyme having ascorbic acid oxidase activity and an enzyme having lipoprotein lipase activity, and specifically contains one or two or more enzymes selected from the group consisting of ascorbic acid oxidase and lipoprotein lipase.

From the viewpoint of avoiding influence of ascorbic acid coexisting in the specimen on measurement accuracy, it is preferable that the first reagent composition further has the ascorbic acid oxidase activity, and it is more preferable that the first reagent composition has the ascorbic acid oxidase activity.

From the same viewpoint, the first reagent composition preferably contains an enzyme having ascorbic acid oxidase activity, and more preferably contains ascorbic acid oxidase.

From the viewpoint of avoiding the influence of ascorbic acid coexisting in the specimen on the measurement accuracy, the ascorbic acid oxidase activity of the first reagent composition is preferably equal to or more than 0.1 U/mL and more preferably equal to or more than 0.2 U/mL, and is preferably equal to or less than 15 U/mL and more preferably equal to or less than 10 U/mL.

The ascorbic acid oxidase activity of the first reagent composition is measured, for example, by the following method. That is, in the measurement of the ascorbic acid oxidase activity, a substrate (10 mM ascorbic acid solution -0.13 mM EDTA is diluted 20 times with 90 mM $KH_2PO_4$ - 5 mM $NaHPO_4$) is used. 1 mL of the substrate is preheated at 30°C, and after 5 minutes, 0.1 mL of the measurement target diluted with a 90 mM $NaHPO_4$ solution containing 0.05% BSA is added and mixed to initiate the reaction. After 5 minutes, 3.0 mL of a reaction stopping solution (0.2N HCl) is added to stop the reaction, and then the absorbance at 245 nm is measured to calculate the ascorbic acid oxidase activity. For example, in a case where the activity amount is equal to or more than 10 U/L, it can be said that the measurement target has ascorbic acid oxidase activity, and more specifically, it can be said that the measurement target contains "ascorbic acid oxidase".

From the viewpoint of preferably adjusting the actions on the various lipoproteins, the lipoprotein lipase activity of the first reagent composition is preferably equal to or more than 2 U/mL and more preferably equal to or more than 50 U/mL, and is preferably equal to or less than 300 U/mL and more preferably equal to or less than 200 U/mL.

The lipoprotein lipase activity of the first reagent composition is measured, for example, by the following method. That is, in the measurement of the lipoprotein lipase activity, an olive oil emulsion solution (a solution obtained by adding 5 mL of 5.0% Triton X100 to 5 g of olive oil and 2 mL of ethanol, subjecting the mixture to sonication for 20 minutes, subsequently adding 25 mL of 4% BSA and 15 mL of a 0.1 M phosphate buffer solution with a pH 7.0 thereto, and carrying out stirring at room temperature for 1 to 2 hours) is used as a substrate solution. A dilution solution (a 20 mM phosphate buffer solution containing 2 mM $MgCl_2$ and 0.5 mM EDTA-2Na; pH: 7.5) is added to the substrate solution preheated at 37°C for 5 minutes so that the concentration thereof is 0.9 to 1.6 U/mL, 0.2 mL of a diluted specimen is added thereto, and after heating at 37°C for 15 minutes, 2.0 mL of a reaction stopping solution (0.2 M trichloroacetic acid) is added thereto. Thereafter, filtration (ADVANTEC No. 131 of Toyo Roshi Kaisha, Ltd. or Whatman No. 42) is carried out to recover the filtrate. To 0.05 mL of the filtrate, 3.0 mL of a color developing reagent (200 mL of a 50 mM MES-NaOH buffer solution, 4 mL of 5% Triton X100, 40 μL of N,N-dimethyl-m-toluidine, 4 mg of 4-aminoantipyrine, 24.2 mg of ATP·2Na·3$H_2O$, 40.7 mg of $MgCl_2$·6$H_2O$, 200 U of glycerol kinase, 500 U of Lα glycerophosphate oxidase, 300 U of peroxidase) is added, the resultant mixed solution is allowed to react at 37°C for 15 minutes, the absorbance at a wavelength of 545 nm is measured, and the lipoprotein lipase activity is calculated. For example, in a case where the amount of change in absorbance is equal to or more than 3 U/L, it can be said that the measurement target has lipoprotein lipase activity, and more specifically, it can be said that lipoprotein lipase is contained.

The lipoprotein lipase is not limited as long as it is an enzyme capable of degrading a lipoprotein, and it is possible to use, for example, a lipoprotein lipase derived from an animal or a microorganism.

In addition, in the present embodiment, the enzymes in the first reagent composition and the second reagent composition described later can also be identified by the following method. That is, first, a fragment peptide obtained by degrading a sample containing a target enzyme with trypsin is detected by a hybrid mass spectrometer. Proteins can be identified by database search (for example, Mascot search) of the mass of peptides obtained using a mass spectrometer and the spectrum (MS/MS data) of fragment ions obtained by colliding with argon gas in the mass spectrometer. When the sequence of the fragment peptide derived from the amino acid sequence in the reagent composition matches with the amino acid sequence registered in the database as a unique sequence, it can be considered that the target enzyme is contained.

The enzymes in the first reagent composition and a second reagent composition described later can be identified, for example, by the following quantification. That is, among the fragment peptides obtained by degrading the target enzyme with trypsin, a peptide that is specific to the target enzyme and gives a strong signal in mass spectrometry is selected as a peptide to be quantified. For the peptide to be quantified, an unlabeled peptide and a peptide labeled with a stable isotope as an internal standard are prepared by chemical synthesis. A sample containing the target enzyme is completely digested with trypsin, a known amount of stable isotope-labeled peptide is added, and the measurement is carried out with a triple quadrupole mass spectrometer (LC-MS/MS) connected to HPLC, in a multiple reaction monitoring mode (MRM mode). A mixed solution of the unlabeled peptide of the peptide to be quantified and the known amount of stable isotope-labeled peptide is measured in the same manner to create a calibration curve of the concentration ratio and the peak area ratio of the internal standard, and the absolute amount of the peptide to be quantified in the sample is calculated. Thus, the target enzyme can be quantified.

(Other components)

The first reagent composition may contain components other than those described above. Examples of the other components include a buffer solution, a salt, a protein having no enzymatic action, a preservative such as sodium azide, a hydrogen donor, and a coupler.

The type of buffer solution can be appropriately selected, for example. Specific examples of the buffer solution include a MOPS (3-morpholinopropane sulfonic acid) buffer solution, a phosphate buffer solution, a Tris buffer solution, a PIPES (piperazine-1,4-bis(2-ethanesulfonic acid)) buffer solution, and a HEPES (4-(2-hydroxyethyl)-1-piperazineethanesulfonic acid) buffer solution.

From the viewpoint of maintaining the enzyme activity in the composition and the viewpoint of improving the storage stability of the reagent, the concentration of the buffer solution is preferably equal to or more than 1 mM, more preferably equal to or more than 5 mM, and still more preferably equal to or more than 10 mM, and is preferably equal to or less than 300 mM, more preferably equal to or less than 200 mM, still more preferably equal to or less than 150 mM, and even still more preferably equal to or less than 100 mM.

Specifically, a salt is formulated as a pH adjuster or an ionic strength adjuster. Specific examples of the salt include basic substances such as sodium salts such as sodium hydroxide and sodium sulfate; potassium salts such as potassium hydroxide; magnesium salts such as magnesium chloride and magnesium sulfate; and ammonium salts such as ammonium sulfate and ammonium chloride. In addition, when the first reagent composition contains at least one of a monovalent cation and a divalent cation, or a salt thereof, the fractionation of sdLDL and L LDL becomes easier.

From the viewpoint of causing the salt to act more stably as a pH adjuster or an ionic strength adjuster, the concentration of the salt in the first reagent composition is preferably equal to or more than 2 mmol/L and more preferably equal to or more than 5 mmol/L, and is preferably equal to or less than 50 mmol/L and more preferably equal to or less than 30 mmol/L with respect to the total composition of the first reagent composition.

From the viewpoint of maintaining the enzyme activity in the composition and the viewpoint of improving the storage stability of the reagent, the pH of the first reagent composition is preferably equal to or more than 6.0 and more preferably equal to or more than 6.5, and is preferably equal to or less than 8.0 and more preferably equal to or less than 7.5.

Specific examples of the protein having no enzymatic action include albumin such as bovine serum albumin (BSA).

From the viewpoint of stabilizing the enzyme in the first reagent composition and the viewpoint of stabilizing the first step of leading the cholesterol in lipoproteins other than sdLDL to the outside of the reaction system, the concentration of albumin such as BSA in the first reagent composition is preferably equal to or more than 1 g/L and more preferably equal to or more than 2 g/L, and is preferably equal to or less than 20 g/L and more preferably equal to or less than 10 g/L with respect to the total composition of the first reagent composition.

The first reagent composition preferably contains at least one of a hydrogen donor or a coupler, and more preferably contains any one of a hydrogen donor or a coupler. At this time, any one of a hydrogen donor or a coupler is used in the first step to lead cholesterol in lipoproteins other than sdLDL to the outside of the reaction system. More specifically, any one of the hydrogen donor or the coupler is used in order to cause the cholesterol esterase or the cholesterol oxidase to act on the cholesterol in the lipoproteins other than sdLDL and convert the generated hydrogen peroxide to colorless quinone in the presence of the peroxidase.

Specific examples of the hydrogen donor include aniline derivatives such as N-ethyl-N-(2-hydroxy-3-sulfopropyl)-3-methylaniline (TOOS), N-ethyl-N-(2-hydroxy-3-sulfopropyl)-3,5-dimethylaniline (MAOS), N-ethyl-N-(3-sulfopropyl)-3-methylaniline (TOPS), N-(2-hydroxy-3-sulfopropyl)-3,5-dimethoxyaniline (HDAOS), N-(3-sulfopropyl)aniline (HALPS), N-(3-sulfopropyl)-3-methoxy-5-aniline (HMMPS), N-ethyl-N-(2-hydroxy-3-sulfopropyl)-4-fluoro-3,5-dimethoxyaniline (FDAOS), and N-ethyl-N-(3-methylphenyl)-N'-succinylethylenediamine (EMSE).

From the viewpoint of leading the cholesterol in the lipoproteins other than sdLDL to the outside of the reaction system, a concentration of the hydrogen donor in the first reagent composition is preferably equal to or more than 1 mM and more preferably equal to or more than 1.5 mM, and is preferably equal to or less than 5 mM and more preferably equal to or less than 3 mM.

In addition, in a case where the hydrogen donor is used in the second reagent composition described later, it is preferable that the first reagent composition contains a coupler used in the coupling reaction. As the coupler, for example, 4-aminoantipyrine (4AA), an aminoantipyrine derivative, vanillindiamine sulfonic acid, methylbenzthiazolinone hydrazone, sulfonated methylbenzthiazolinone hydrazone, and the like can be used, and the coupler is not limited thereto.

The concentration of the coupler in the first reagent composition is preferably equal to or more than 0.2 mM, and more preferably equal to or more than 0.3 mM, and is preferably equal to or less than 5.0 mM and more preferably equal to or less than 3.3 mM with respect to the total composition of the reaction solution after the addition of the first reagent composition from the viewpoint of causing the coupler to stably act on sdLDL.

(Kit)

In the present embodiment, the kit is used for quantifying sdLDL-C in a sample, and contains the above-mentioned first reagent composition and a second reagent composition for quantifying sdLDL-C.

In addition, the kit is specifically used for a method of quantifying sdLDL-C including two or more steps. At this time, each of the first and second reagent compositions is used in different steps, and the first and second reagent compositions are preferably used in this order.

Hereinafter, the configuration of the second reagent composition will be described in more detail.

(Second reagent composition)

The second reagent composition is specifically a reagent composition for quantifying sdLDL-C. Although the components of the second reagent composition differ depending on the configuration of the first reagent composition, any formulation composition capable of quantifying sdLDL-C may be used, and known substances can be used.

Specific examples of the component contained in the second reagent composition include an enzyme, a buffer solution, a salt, a surfactant, a protein having no enzymatic action, a preservative, and any one of a hydrogen receiver and donor or a coupler.

In addition, in a preferable configuration of the kit, the first reagent composition contains any one of a hydrogen donor or a coupler and does not contain the other, and the second reagent composition does not contain any one of the hydrogen donor or the coupler and contains the other.

Examples of the enzyme include peroxidase. In addition, for example, the second reagent composition has peroxidase activity. From the viewpoint of accurately measuring the sdLDL-C in the second step, the peroxidase activity of the second reagent composition is preferably equal to or more than 500 U/L and more preferably equal to or more than 1000 U/L, and is preferably equal to or less than 10000 U/L. Since the peroxidase activity is exhibited over the second step in a case where the first reagent composition has the peroxidase activity, it is also possible to reduce the concentration or make the peroxidase not contained in the second reagent.

The types of buffer solution and salt can be appropriately selected depending on, for example, the type of enzyme contained in the second reagent composition. Specific examples of the buffer solution include those described above for the first reagent composition.

From the viewpoint of maintaining the enzyme activity in the composition and the viewpoint of improving the storage stability of the reagent, the pH of the second reagent composition is preferably equal to or more than 6.0 and more preferably equal to or more than 6.5, and is preferably equal to or less than 8.0 and more preferably equal to or less than 7.5.

Examples of the surfactant include surfactants that act on sdLDL. In addition, the second reagent composition preferably contains a surfactant that acts on sdLDL from the viewpoint of stably quantifying sdLDL-C.

The surfactant that acts on sdLDL may be a surfactant that selectively acts on sdLDL, such as a surfactant that acts only on sdLDL, or a surfactant that also acts on lipoproteins other than sdLDL, or a surfactant that acts on all lipoproteins.

As the surfactant in the second reagent composition, for example, a polyoxyethylene derivative can be mentioned, and any commercially available surfactant can be used as long as the surfactant is used in reagents for total cholesterol measurement, or the like. Examples of such a surfactant include polyoxyethylene alkyl phenyl ether such as polyoxyethylene octylphenyl ether (for example, Emulgen 909 (manufactured by Kao Corporation), and Triton X-100) and polyoxyethylene alkyl ether (for example, Emulgen 707 and Emulgen 709 (manufactured by Kao Corporation)) .

The concentration of the surfactant in the second reagent composition is preferably equal to or more than 0.05% (w/v), more preferably equal to or more than 0.1% (w/v), and still more preferably equal to or more than 0.5% (w/v) with respect to the mixed solution after the addition of the second reagent composition from the viewpoint of causing the surfactant to stably act on sdLDL.

In addition, from the same viewpoint, the concentration of the surfactant in the second reagent composition is preferably equal to or less than 8.0% (w/v), and more preferably equal to or less than 5.0% (w/v).

In a case where the second reagent composition contains a coupler, the coupler is preferably one or two or more compounds selected from the group consisting of 4-aminoantipyrine (4-AA), an aminoantipyrine derivative, vanillindiamine sulfonic acid, methylbenzthiazolinone hydrazone, and sulfonated methylbenzthiazolinone hydrazone.

The concentration of the coupler in the second reagent composition is preferably equal to or more than 0.5 mM, and more preferably equal to or more than 1.0 mM, and is preferably equal to or less than 15 mM and more preferably equal to or less than 10 mM with respect to the total composition of the reaction solution after the addition of the second reagent composition from the viewpoint of causing the coupler to stably act on sdLDL.

On the other hand, in a case where the coupler is contained in the first reagent composition, the hydrogen donor is preferably contained in the second reagent composition. Specific examples of the hydrogen donor include those described above for the first reagent composition. In this case, the concentration of the hydrogen donor in the reagent is preferably equal to or more than 3 mM, and more preferably equal to or more than 4.5 mM, and is preferably equal to or less than 15 mM and more preferably equal to or less than 12 mM with respect to the total composition of the reaction solution after the addition of the second reagent composition from the viewpoint of causing the hydrogen donor to stably act on sdLDL.

Specific examples of each of the proteins having no enzymatic action and the preservative include those described above for the first reagent composition.

(Method)

A method in the present embodiment is a method of quantifying sdLDL-C in a sample using the above-mentioned first and second reagent compositions. The quantification method in the present embodiment includes the following first step and second step.

(First Step) Causing the above-described first reagent composition to react to the sample.

(Second Step) After the first step, causing the above-described second reagent composition to react, thereby quantifying cholesterol in a remaining lipoprotein.

The configurations of the first and second reagent compositions are as described above. In such a method, by using the first reagent composition in the present embodiment, it is possible to perform a measurement with excellent accuracy after specimen dilution and specificity. Therefore, for example, it is also possible to stably perform the quantification of the sdLDL-C as a reagent having good dilution linearity and high correlation with the reference method.

In addition, the method of quantifying sdLDL-C may be carried out by, for example, adding the first reagent composition to a sample (test sample) to allow a reaction to proceed, then adding the second reagent composition to the sample to allow a reaction to proceed, and measuring the absorbance.

The test sample is, for example, a sample derived from blood such as serum or plasma, and preferably serum.

The first and second steps are usually performed in an automatic analyzer.

The amount of the sample and the amount of each reagent composition can be appropriately determined in consideration of, for example, the concentration of the reagent in each reagent composition, and the amounts are determined within a range applicable to the automatic analyzer. For example, 1 to 10 $\mu$L of the test sample, 50 to 300 $\mu$L of the first reagent, and 25 to 200 $\mu$L of the second reagent may be used.

Each step will be described in more detail below.

(First Step)

In the first step, the first reagent composition is allowed to act on a sample. By doing this, lipoproteins other than sdLDL are eliminated, and cholesterol in the lipoproteins other than sdLDL is liberated and led to the outside of the reaction system.

More specifically, in the first step, preferably, a surfactant that acts on lipoproteins other than the sdLDL is allowed to act on the sample in the presence of sphingomyelinase and cholesterol esterase. Then, cholesterol generated by liberation from lipoproteins is allowed to react with an enzyme that reacts with cholesterol, such as cholesterol oxidase, and is led to the outside of the reaction system. In the first step, for example, known technologies for eliminating cholesterol in lipoproteins other than sdLDL and leading the cholesterol to the outside of the reaction system, aggregating cholesterol in lipoproteins other than sdLDL, inhibiting cholesterol in lipoproteins other than sdLDL to avoid the reaction in the subsequent step, and the like can be used.

When the first reagent composition has an electron donor, the eliminating cholesterol generated from lipoproteins other than sdLDL in the first step and leading the cholesterol to the outside of the reaction system may include, for example, forming a colorless quinone in the presence of hydrogen peroxide generated by sphingomyelinase, cholesterol esterase activity, and cholesterol oxidase activity in the first reagent composition and of an electron donor.

In addition, in the first step, at least one of a monovalent cation and a divalent cation or a salt thereof can be used as an ionic strength adjuster by further adding such ionic strength adjuster to the reaction solution. Addition of such ionic strength adjuster facilitates differentiation of sdLDL and L LDL.

(Second Step)

In the second step, the sdLDL-C remaining after the first step is quantified. In the second step, an LDL quantification method used in the related art can be used. Examples thereof include a method of quantifying the content of an LDL specific aggregate formed by adding an LDL aggregating agent according to turbidimetry, a method of using an antigen-antibody reaction with an LDL specific antibody, a method of quantifying a degraded product using an enzyme. The quantification method is selected, for example, according to the components or the composition contained in the second reagent composition.

When the second reagent composition contains an enzyme, a method of quantifying a degraded product using an enzyme can be adopted. Specifically, the second reagent composition containing, for example, one or two or more enzymes for measuring cholesterol, selected from the group consisting of cholesterol esterase, cholesterol oxidase, cholesterol dehydrogenase, and peroxidase is added to the reaction solution after the first step, to liberate and degrade sdLDL-C and quantifying the reaction product thereof.

In the present embodiment, the reaction temperature in each step is preferably 2°C to 45°C, and more preferably 25°C to 40°C.

Regarding the reaction time, each step is preferably carried out for 1 to 10 minutes and more preferably carried out for 3 to 7 minutes.

Examples of the automated analyzer to be used for quantifying sdLDL-C include TBA-120FR and TBA-200FR (all manufactured by TOSHIBA Corporation), JCA-BM 1250, JCA-BM 1650, and JCA-BM 2250 (all manufactured by JEOL Ltd.), HITACHI 7180, and HITACHI 7170 (all manufactured by Hitachi, Ltd.), AU2700, AU5800, and AU680 (all manufactured by Olympus Corporation), and cobas c501 and cobas c701 (all manufactured by Roche).

The sdLDL-C is quantified by measuring the absorbance in a wavelength range of 580 to 720 nm, preferably 600 to 700 nm, for example.

Although the embodiments of the present invention have been described above, these are examples of the present invention, and various configurations other than the above can be adopted. Examples

(Examples 1 to 7 and Comparative Examples 1 and 2)

A first reagent composition of each example, in which the contact angle was changed, was prepared. Then, the first reagent composition was combined with the second reagent composition to perform the quantification of the sdLDL-C, so that the accuracy after the specimen dilution for measurement and specificity (correlation with the reference method) were evaluated.

(Preparation of reagent composition)

Each component shown below was formulated at the following concentration, and the first reagent composition and the second reagent composition of each example were prepared.

For the first reagent composition, the composition of each example was prepared by changing the type and concentration of the nonionic surfactant.

The formulation composition of the first reagent composition and the second reagent composition used for the measurement are shown below.

(First reagent composition)

| | |
|---|---|
| PIPES buffer solution, pH 7.0 | 50 mM |
| Cholesterol esterase | 300 U/L |
| Cholesterol oxidase | 600 U/L |
| Sphingomyelinase | 2700 U/L |
| Catalase | 1200 KU/L |
| Bovine serum albumin | 1.0% (w/v) |
| TOOS | 2.0 mM |
| Nonionic surfactant *1 | |

*1 Nonionic surfactant: Polyoxyethylene benzylphenyl ether derivative and polyoxyethylene styrenated phenyl ether derivative were used, and the added amounts thereof were changed to adjust the first reagent compositions a to i.

(Second reagent composition)

| | |
|---|---|
| PIPES buffer solution, pH 7.0 | 50 mM |
| 4-aminoantipyrine | 4.0 mM |
| Peroxidase | 4000 U/L |
| Sodium azide | 0.05% (w/v) |
| Polyoxyethylene octylphenyl ether | 1% (w/v) |

(Measurement of sdLDL-C)

sdLDL-C was measured using the first reagent composition and the second reagent composition of each example. Specifically, in each example, that is, the first reagent compositions having different contact angles and the second reagent composition were prepared. 150 μL of the first reagent composition was added to 3 μL of the serum sample and reacted at 37°C for 5 minutes, then, 50 μL of the second reagent composition was added thereto and reacted for 5 minutes to measure the absorbance at a main wavelength of 600 nm and a minor wavelength of 700 nm. Then, the concentration of sdLDL-C was calculated by a calibration curve.

(Measurement method of contact angle)

As a PET base material, a visually transparent PET (amorphous polyester) resin plate (manufactured by C.I. TAKIRON Corporation, PET plate (PETEC) product number 6010, thickness: 2 mm) was used. A surface of the unused PET base material was wiped with a 70% ethanol aqueous solution, and then the measurement was performed within 5 minutes.
Using a contact angle meter (manufactured by Kyowa Interface Science Co., Ltd., DMo-501), the contact angle was measured 1 second after the dropwise addition under the conditions of a temperature of 15°C to 25°C, a syringe of Teflon-coated 18G, a dropwise addition method, and a dropwise addition amount of 2 μL by a liquid droplet method and a θ/2 method.
The measurement was performed 5 times for each example, and the average value of the 5 times was calculated. The measurement results are shown in Table 1.

(Measurement method of viscosity)

For Examples and Comparative Examples, the viscosity of the first reagent composition in each example was measured by an electromagnetic spinning method using an EMS viscometer (EMS-1000 manufactured by KYOTO ELECTRONICS MANUFACTURING Co., LTD.) at a motor rotation speed of 1,000 rpm, a holding time of 600 seconds, a measurement temperature of 5°C and 37°C, a sequence loop of 1 time, and a sample of 500 μL. For each example, the measurement was performed 5 times at each temperature, and the average value of the 5 times was calculated. The measurement results are shown in Table 1.

(Accuracy test after specimen dilution)

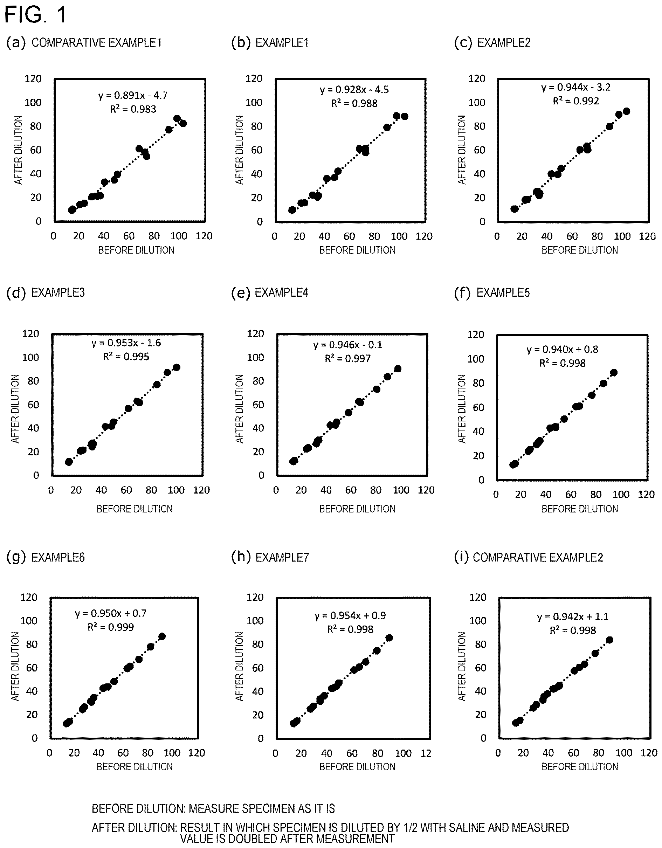

Human serum with known sdLDL-C concentrations at each concentration was diluted twofold with physiological saline, and the sdLDL-C concentration in each example before and after the dilution was measured. The specimen measurement value after dilution was multiplied by a dilution factor of 2 and compared with the measurement value before dilution, thereby calculating a primary regression equation.
A summary of the measurement results is shown in Table 1. In addition, detailed data of each example is shown in Fig. 1(a) to Fig. 1(i) . In Fig. 1(a) to Fig. 1(i), a horizontal axis represents the specimen measurement value before dilution, and a vertical axis represents the measurement value after the specimen dilution, obtained by multiplying by a dilution ratio of 2. The evaluation criteria are described below.

A: A slope of the primary regression equation was within $1.00 \pm 0.06$ (0.94 to 1.06), and a correlation coefficient r (the same applies hereinafter) of the primary regression equation was

$$r > 0.9 \ (r^2 > 0.81)$$

B: The slope of the primary regression equation was more than $1.00 \pm 0.06$ and within $1.00 \pm 0.10$, and $r > 0.9$ ($r^2 > 0.81$)
C: The slope of the primary regression equation was more than $1.00 \pm 0.10$, or $r \leq 0.9$ ($r^2 \leq 0.81$)

From Table 1 and Fig. 1(a) to Fig. 1(i), good accuracy after the specimen dilution was obtained in each example.

(Correlation test with reference method)

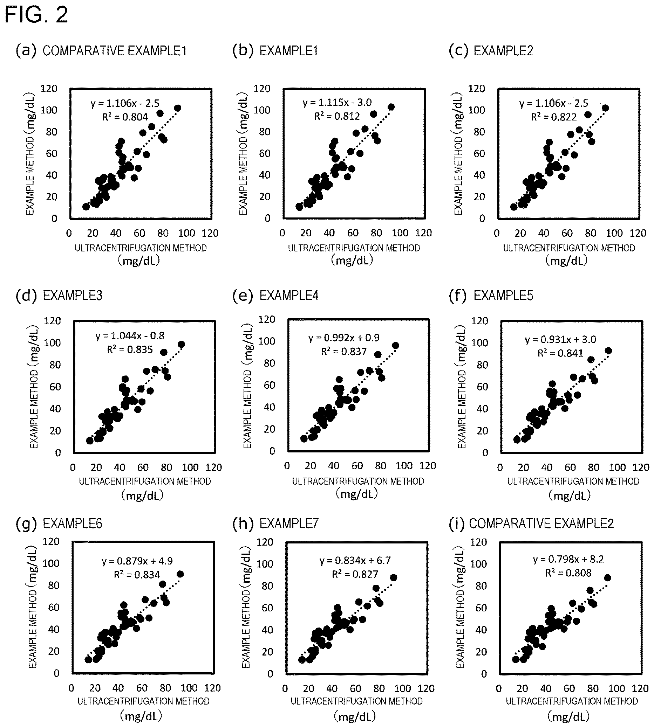

As a comparative target method, a value of the ultracentrifugation method sdLDL-C (specific gravity: 1.044 to 1.063) in each human specimen was calculated and compared with the sdLDL-C value of each example.
A summary of the measurement results is shown in Table 1. In addition, detailed correlation data of each example is shown in Fig. 2(a) to Fig. 2(i). A horizontal axis of Fig. 2(a) to Fig. 2(i) shows the sdLDL-C value calculated by the ultracentrifugation method, which is the reference method, and a vertical axis shows the sdLDL-C value in each example. The evaluation criteria are described below. The following evaluations of A and B were passed.

A: A slope was within $1.00 \pm 0.11$ (0.89 to 1.11), and the correlation coefficient r of the primary regression equation (the same applies hereinafter.) > 0.905 ($r^2$ > 0.819).
B: The slope was more than $1.00 \pm 0.11$ and within $1.00 \pm 0.20$, and r > 0.900 ($r^2$ > 0.810) .
C: The slope was more than $1.000 \pm 0.200$, or $0.900 \leq$ r ($r^2 \leq 0.81$) .

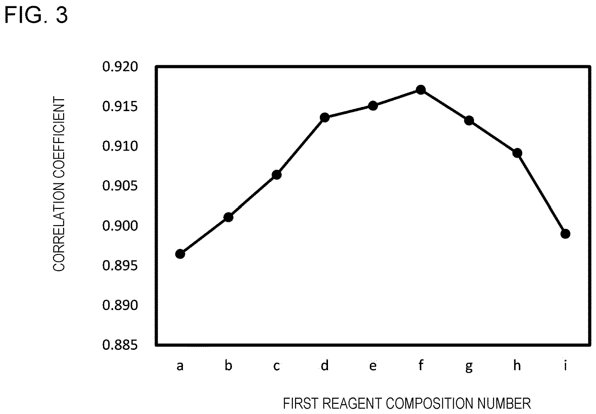

From Table 1 and Fig. 2(a) to Fig. 2(i), a good correlation with the ultracentrifugation method was obtained in each example.
In addition, Fig. 3 is a diagram showing a relationship with the correlation coefficient when the reagent compositions are arranged in order from a reagent composition having a large contact angle to a reagent composition having a small contact angle. The results showed that the correlation coefficient was higher in the contact angles of reagent compositions b to h.

(Dilution linearity test)

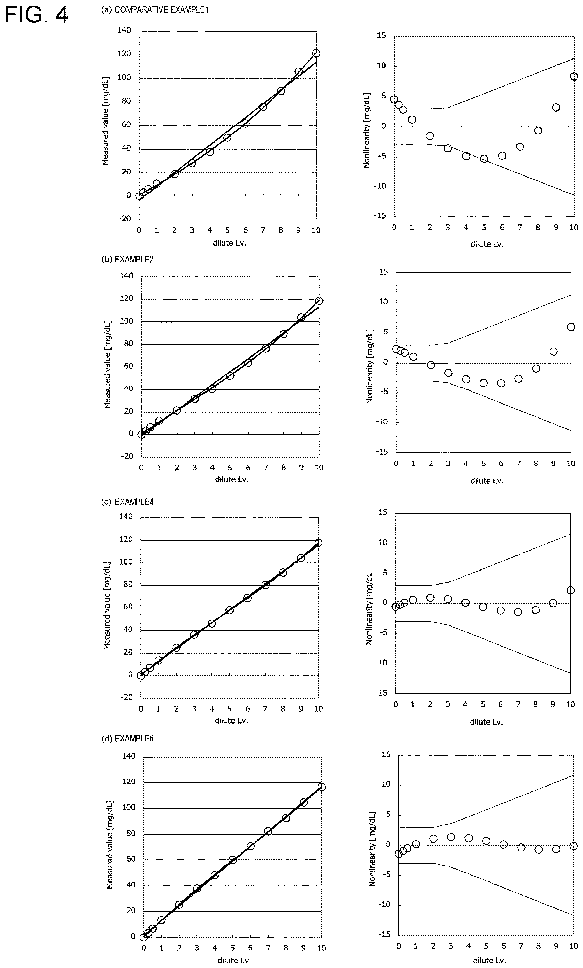

Among the examples shown in Table 1, a dilution linearity test was performed on Comparative Example 1 and Examples 2, 4, and 6. Specifically, a high-concentration sdLDL-C specimen with a known concentration was diluted in physiological saline in 10 stages, and the sdLDL-C concentration of each example in each dilution series was measured. The analysis was performed according to the CLSI guideline EP06-A, and a difference plot of nonlinearity was calculated to evaluate the dilution linearity.
Table 1 shows a summary of the measurement results, and detailed data is shown in Fig. 4(a) to Fig. 4(d). In the left figures of Fig. 4(a) to Fig. 4(d), a horizontal axis (dilute Lv.) indicates a dilution series of a high-concentration specimen, and a vertical axis (Measured value [mg/dL]) indicates an sdLDL-C value. In addition, in the right figures of Fig. 4(a) to Fig. 4(d), a horizontal axis (dilute Lv.) indicates a dilution series of the high-concentration specimen, and a vertical axis (Nonlinearity [mg/dL]) indicates non-linearity of the sdLDL-C value. The evaluation criteria were set as follows in accordance with the CLSI guideline EP06-A. The following evaluations of A and B were passed.

A: A nonlinearity value (circular plot in the right figure of Fig. 4) was within an allowable range (inside the two solid lines in the right figure of Fig. 4: $\pm 3$ mg/dL in a case of Linear fit sdLDL-C < 30 mg/dL, $\pm 10\%$ in a case of Linear fit sdLDL-C $\geq$ 30 mg/dL) at all points
C: A nonlinearity value (circular plot in the right figure of Fig. 4) is outside the allowable range (inside the two solid lines in the right figure of Fig. 4: $\pm 3$ mg/dL in the case of linear fit sdLDL-C < 30 mg/dL, $\pm 10\%$ in the case of linear fit sdLDL-C $\geq$ 30 mg/dL) at least one point

From Table 1 and Fig. 4(a) to Fig. 4(d), good dilution linearity was obtained in each example.
From Fig. 1(a) to Fig. 1(i), Fig. 2(a) to Fig. 2(i), Fig. 3, Fig. 4(a) to Fig. 4(d), and Table 1, in each example, it was possible to measure the sdLDL-C with excellent dilution linearity, accuracy after specimen dilution, and correlation with the reference method.

[Table 1]

Table 1

| | | Comparative Example 1 | Example 1 | Example 2 | Example 3 | Example 4 | Example 5 | Example 6 | Example 7 | Comparative Example 2 |
|---|---|---|---|---|---|---|---|---|---|---|
| First reagent composition number | | a | b | c | d | e | f | 9 | h | i |
| Contact angle (°) | | 67.4 | 66.9 | 66.0 | 65.3 | 64.9 | 64.0 | 63.5 | 63.2 | 62.8 |
| Viscosity (5°C)(mPa·s) | | 1.89 | 2.06 | 1.87 | 1.89 | 1.95 | 1.99 | 1.96 | 2.17 | 1.96 |
| Viscosity (37°C) (mPa·s) | | 1.09 | 0.86 | 0.93 | 0.91 | 0.88 | 0.92 | 1.01 | 0.93 | 0.95 |
| Evaluation | Accuracy after specimen dilution | C | B | A | A | A | A | A | A | A |
| | Correlation with reference method | C | B | A | A | A | A | B | B | C |
| | Linearity | C | - | A | - | A | - | A | - | - |

EP 4 509 838 A1

...

(Examples 8 to 10)

(Preparation of reagent composition)

Each component shown below was formulated at the following concentration, and the type of surfactant was changed to prepare the first reagent composition of each example. The formulation composition of the first reagent composition is shown below.

(First reagent composition)

| PIPES buffer solution, pH 7.0 | 50 mM |
|---|---|
| Cholesterol esterase | 900 U/L |
| Cholesterol oxidase | 450 U/L |
| Sphingomyelinase | 525 U/L |
| Bovine serum albumin | 0.75% (w/v) |
| Catalase | 1200 KU/L |
| 4AA | 1 mM |
| Surfactant *2 | |
| *2 Surfactant | |

Example 8: POE monostyrenated phenyl ether, a degree of polymerization of POE of 11 to 33
Example 9: POE monostyrenated phenyl ether, a degree of polymerization of POE of 13 to 37
Example 10: polyoxyethylene benzylphenyl ether derivative

(Evaluation of lipoprotein selectivity)

In order to evaluate the lipoprotein selectivity in the reaction with the first reagent composition, the reactivity of sdLDL and lbLDL with the above-mentioned reagent composition was measured, respectively.

Specifically, sdLDL and lbLDL separated from human serum by ultracentrifugation were used as specimens. The first reagent composition was prepared to confirm the reactivity of the surfactant with the specimen. When evaluation is performed, in order to easily perform the evaluation by causing a color development reaction only with the first reagent composition to measure the absorbance, a composition in which 1.5 mM TOOS and 1250 U/L of peroxidase were formulated instead of 1200 KU/L of catalase in the above-described composition was used as the first reagent composition for evaluation of each example.

3 μL of the specimen and 150 μL of first reagent composition in each example were mixed, and a difference in absorbance at 600 nm (main wavelength) and 700 nm (minor wavelength) after 5 minutes at 37°C (In Table 2, "absorbance at wavelength 600 nm/700 nm") [mAbs] was measured to obtain the ratio of the absorbance of lbLDL with respect to the absorbance of sdLDL. The measurement results are shown in Table 2.

[Table 2]

Table 2

| | Example 8 | Example 9 | Example 10 |
|---|---|---|---|
| Contact angle | 63.8 | 65.5 | 66.1 |
| POE monostyrenated phenyl ether (%) | 0.17 | 0.17 | 0 |
| lbLDL absorbance (at wavelength 600 nm/700 nm) | 2563 | 3391 | 2911 |
| sdLDL absorbance (at wavelength 600 nm/700 nm) | 1761 | 1768 | 2493 |
| lbLDL/sdLDL absorbance ratio | 1.46 | 1.92 | 1.17 |

From Table 2, the sdLDL in the sample can be more accurately quantified by using the first reagent composition of each Example. In addition, from Table 2, it is shown that, even in each example, the first reagent composition containing POE monostyrenated phenyl ether has an absorbance ratio of LDL, which is further excellent in selectivity of lbLDL to LDL in the first step.

(Comparative Example 3)

(Preparation of reagent composition)

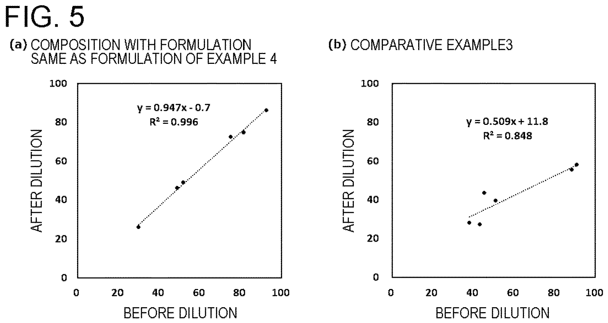

The first reagent composition containing polyoxyethylene styrenated phenyl ether sulfate as an anionic surfactant was prepared according to the above-described method in Examples 1 to 7 and Comparative Examples 1 and 2, and the contact angle and viscosity were measured according to the method described in the above-described examples, so that the accuracy after the specimen dilution was evaluated. In the measurement of the accuracy after the specimen dilution, in addition to the first reagent composition of Comparative Example 3, the first reagent composition having the same formulation as in Example 4 described above was prepared and measured by contrast. The measurement results are shown in Table 3. Table 3 shows the measurement results of the contact angle and the viscosity of Example 4 described above (Table 1). In addition, detailed data is shown in Fig. 5(a) and Fig. 5(b). Fig. 5(a) and Fig. 5(b) show the measurement results of the first reagent composition with the same formulation as that in Example 4 and Comparative Example 3, respectively.

[Table 3]

Table 3

|  |  | Example 4 | Comparative Example 3 |
|---|---|---|---|
| Contact angle (°) |  | 64.9 | 68.8 |
| Viscosity (5°C) (mPa·s) |  | 1.95 | 1.62 |
| Viscosity (37°C)(mPa·s) |  | 0.88 | 0.53 |
| Evaluation | Accuracy after specimen dilution | A | C |

From Fig. 5(b), in Comparative Example 3, in which the anionic surfactant was used, the sdLDL could not be accurately quantified after specimen dilution in the sample. In contrast, in the formulation (Fig. 5(a)) of Example 4, in which the nonionic surfactant was used, the sdLDL could be accurately quantified even after specimen dilution. In addition, from Fig. 5(a) and Fig. 1(e), it was confirmed that the composition with the same formulation could evaluate the selectivity after the specimen dilution with excellent reproducibility.

The present application claims priority based on Japanese Patent Application No. 2022-064966 filed on April 11, 2022, the entire content of which is incorporated herein by reference.

**Claims**

1. A reagent composition for use as a first reagent composition in a method of quantifying a small dense LDL cholesterol (sdLDL-C) in a sample, the method including

    causing the first reagent composition to react to the sample, and
    after the causing the first reagent composition to react to the sample, causing a second reagent composition for quantifying the sdLDL-C to react, thereby quantifying cholesterol in a remaining lipoprotein,
    the reagent composition comprising a nonionic surfactant and having cholesterol esterase activity, cholesterol oxidase activity, and sphingomyelinase activity, wherein:

      a contact angle between the reagent composition and a polyethylene terephthalate (PET) base material, which is measured by the following method 1, is equal to or more than 63.0° and equal to or less than 67.0°,
      (method 1)

(1) the PET base material: PET (amorphous polyester) resin plate, transparent, thickness of 2 mm,
(2) a pre-treatment: a surface of an unused PET base material is wiped with a 70% ethanol aqueous solution, and a measurement is performed within 5 minutes after the wiping,
(3) a measurement method and conditions: a liquid droplet method, a $\theta/2$ method, a temperature: 15°C to 25°C, a syringe: Teflon-coated 18G, a dropwise addition method, a dropwise addition amount: 2 $\mu$L, a measurement 1 second after the dropwise addition, and
(4) a calculation of the contact angle: the measurement is performed 5 times, and an average value of the 5 times is obtained.

2. The reagent composition according to Claim 1,

wherein a viscosity of the reagent composition at 5°C, which is measured by the following method 2-1, is equal to or less than 2.5 mPa-s,
(method 2-1)

(1) a device: an electro magnetically spinning (EMS) viscometer,
(2) a measurement method and conditions: a measurement type: an electromagnetic spinning method, a motor rotation speed: 1,000 rpm, a holding time: 600 seconds, a measurement temperature: 5°C, a sequence loop: 1 time, a sample: 500 $\mu$L, and
(3) a calculation of the viscosity: the measurement is performed 5 times, and an average value of the 5 times is calculated.

3. The reagent composition according to Claim 1 or 2,

wherein a viscosity of the reagent composition at 37°C, which is measured by the following method 2-2, is equal to or less than 1.05 mPa·s,
(method 2-2)

(1) a device: an electro magnetically spinning (EMS) viscometer,
(2) a measurement method and conditions: a measurement type: an electromagnetic spinning method, a motor rotation speed: 1,000 rpm, a holding time: 600 seconds, a measurement temperature: 37°C, a sequence loop: 1 time, a sample: 500 $\mu$L, and
(3) a calculation of the viscosity: the measurement is performed 5 times, and an average value of the 5 times is calculated.

4. The reagent composition according to Claim 1 or 2,
wherein the nonionic surfactant contains polyoxyethylene monostyrenated phenyl ether.

5. The reagent composition according to Claim 4,
wherein a degree of polymerization n of polyoxyethylene in the polyoxyethylene monostyrenated phenyl ether is equal to or more than 5 and equal to or less than 80.

6. The reagent composition according to Claim 4,
wherein a content of the polyoxyethylene monostyrenated phenyl ether in the reagent composition is equal to or more than 0.05% (w/v) and equal to or less than 0.6% (w/v) with respect to an entire reagent composition.

7. The reagent composition according to Claim 1 or 2,
wherein the reagent composition contains any one of a hydrogen donor or a coupler.

8. The reagent composition according to Claim 1 or 2,
wherein the reagent composition further has ascorbic acid oxidase activity.

9. The reagent composition according to Claim 1 or 2,
wherein the reagent composition further has at least one activity selected from the group consisting of peroxidase activity and catalase activity.

10. A kit used for quantifying a sdLDL-C in the sample, the kit comprising:

a first reagent composition consisting of the reagent composition according to Claim 1 or 2; and a second reagent composition for quantifying the sdLDL-C.

11. The kit according to Claim 10,
wherein the second reagent composition has peroxidase activity.

# FIG. 1

(a) COMPARATIVE EXAMPLE1

y = 0.891x - 4.7
$R^2$ = 0.983

(b) EXAMPLE1

y = 0.928x - 4.5
$R^2$ = 0.988

(c) EXAMPLE2

y = 0.944x - 3.2
$R^2$ = 0.992

(d) EXAMPLE3

y = 0.953x - 1.6
$R^2$ = 0.995

(e) EXAMPLE4

y = 0.946x - 0.1
$R^2$ = 0.997

(f) EXAMPLE5

y = 0.940x + 0.8
$R^2$ = 0.998

(g) EXAMPLE6

y = 0.950x + 0.7
$R^2$ = 0.999

(h) EXAMPLE7

y = 0.954x + 0.9
$R^2$ = 0.998

(i) COMPARATIVE EXAMPLE2

y = 0.942x + 1.1
$R^2$ = 0.998

BEFORE DILUTION: MEASURE SPECIMEN AS IT IS

AFTER DILUTION: RESULT IN WHICH SPECIMEN IS DILUTED BY 1/2 WITH SALINE AND MEASURED
VALUE IS DOUBLED AFTER MEASUREMENT

# FIG. 2

(a) COMPARATIVE EXAMPLE1

(b) EXAMPLE1

(c) EXAMPLE2

(d) EXAMPLE3

(e) EXAMPLE4

(f) EXAMPLE5

(g) EXAMPLE6

(h) EXAMPLE7

(i) COMPARATIVE EXAMPLE2

FIG. 3

FIG. 4

(a) COMPARATIVE EXAMPLE1

(b) EXAMPLE2

(c) EXAMPLE4

(d) EXAMPLE6

# FIG. 5

(a) COMPOSITION WITH FORMULATION
SAME AS FORMULATION OF EXAMPLE 4

(b) COMPARATIVE EXAMPLE3

$y = 0.947x - 0.7$
$R^2 = 0.996$

$y = 0.509x + 11.8$
$R^2 = 0.848$

| INTERNATIONAL SEARCH REPORT | International application No. |
|---|---|
| | **PCT/JP2023/014549** |

**A.    CLASSIFICATION OF SUBJECT MATTER**

*G01N 33/92*(2006.01)i; *C12Q 1/26*(2006.01)i; *C12Q 1/34*(2006.01)i; *C12Q 1/44*(2006.01)i
FI:    G01N33/92 A; C12Q1/44; C12Q1/26; C12Q1/34

According to International Patent Classification (IPC) or to both national classification and IPC

**B.    FIELDS SEARCHED**

Minimum documentation searched (classification system followed by classification symbols)

G01N33/92; C12Q1/26; C12Q1/34; C12Q1/44

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y | JP 2021-040520 A (DENKA CO LTD) 18 March 2021 (2021-03-18)<br>entire text, all drawings, in particular, see claims, paragraphs [0033], [0071]-[0109], etc. | 1-11 |
| Y | JP 2022-026970 A (DENKA CO LTD) 10 February 2022 (2022-02-10)<br>entire text, all drawings, in particular, see claims, paragraphs [0091], [0092], etc. | 1-11 |
| Y | JP 2007-325587 A (FUJIFILM CORP) 20 December 2007 (2007-12-20)<br>entire text, all drawings, in particular, see claims, paragraphs [0029]-[0031], [0078], fig. 9, etc. | 4-5 |
| A | WO 2008/105486 A1 (DENKA SEIKEN CO., LTD.) 04 September 2008 (2008-09-04)<br>see entire text, all drawings, etc. | 1-11 |
| A | WO 2007/026829 A1 (DENKA SEIKEN CO., LTD.) 08 March 2007 (2007-03-08)<br>see entire text, all drawings, etc. | 1-11 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

| International application No. |
| --- |
| **PCT/JP2023/014549** |

| Patent document cited in search report | | | Publication date (day/month/year) | Patent family member(s) | Publication date (day/month/year) |
| --- | --- | --- | --- | --- | --- |
| JP | 2021-040520 | A | 18 March 2021 | US 2022/0299535 A1 entire text, all drawings, in particular, see claims, paragraphs [0107], [0148]-[0198], etc. WO 2021/049518 A1 EP 4029948 A1 | |
| JP | 2022-026970 | A | 10 February 2022 | (Family: none) | |
| JP | 2007-325587 | A | 20 December 2007 | US 2007/0275429 A1 entire text, all drawings, in particular, see claims, paragraphs [0048]-[0050], [0102], fig. 9, etc. EP 1854894 A1 | |
| WO | 2008/105486 | A1 | 04 September 2008 | US 2010/0035288 A1 see entire text, all drawings, etc. JP 2013-148589 A EP 2116612 A1 | |
| WO | 2007/026829 | A1 | 08 March 2007 | US 2009/0263844 A1 see entire text, all drawings, etc. JP 2012-165761 A EP 1930442 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000325097 A

- JP 2022064966 A

**Non-patent literature cited in the description**

- *JAMA*, 1988, vol. 260, 1917-21
- The Fats of Life Summer 2002, LVDD 15 YEAR ANNIVERSARY ISSUE. HANDBOOK OF LIPO-PROTEIN TESTING. AACC PRESS, vol. AVI, 15-16

- *Atherosclerosis*, 1994, vol. 106, 241-253
- *Atherosclerosis*, 1990, vol. 83, 59